# FASCICULE DE BREVET EUROPEEN

(11) **EP 2 342 557 B1**
(45) Date de publication et mention de la délivrance du brevet: **10.09.2014**
(21) Numéro de dépôt: 09753145.3
(22) Date de dépôt: 27.10.2009
(51) Int. Cl.: G01N 31/12, G01N 33/24

(54) **MÉTHODE ET DISPOSITIF POUR LA CARACTÉRISATION ET LA QUANTIFICATION RAPIDES DU SOUFRE DANS DES ROCHES SÉDIMENTAIRES ET DANS DES PRODUITS PÉTROLIERS**
VERFAHREN UND VORRICHTUNG ZUR RASCHEN CHARAKTERISIERUNG UND QUANTIFIZIERUNG VON SCHWEFEL IN FELSSEDIMENTEN UND ERDÖLPRODUKTEN
METHOD AND DEVICE FOR RAPIDLY CHARACTERISING AND QUANTIFYING SULFUR IN ROCK SEDIMENTS AND PETROL PRODUCTS

(30) Priorité: 29.10.2008 FR 0806015
(43) Date de publication de la demande: 13.07.2011
(73) Titulaire: IFP Energies nouvelles, 92852 Rueil-Malmaison Cedex (FR); Vinci Technologies, 92022 Nanterre (FR)
(72) Inventeur: ESPITALIE, Jean, F-78110 Le Vésinet (FR); ANTONAS, Roland, F-92400 Courbevoie (FR); LAMOUREUX-VAR, Violaine, F-78400 Chatou (FR); LETORT, Gérémie, F-78520 Limay (FR); PILLOT, Daniel, F-78400 Chatou (FR); BEAUMONT, Valérie, F-93100 Montreuil-sous-Bois (FR); HAESELER,Frank, F-92250 La Garenne Colombes (FR)
(86) Numéro de dépôt international: PCT/FR2009/001253
(87) Numéro de publication internationale: WO 2010/049609

(56) Documents cités:
- EP-A- 0 767 375
- WO-A-2005/111603
- FR-A- 2 376 414
- GB-A- 1 461 518

## Description

Le domaine d'application de cette invention concerne une méthode et un dispositif pour effectuer des mesures de quantification du soufre dans des échantillons de sédiments, de produits pétroliers ou autres matériaux. Elle permet en particulier de caractériser et de quantifier le soufre dans des roches sédimentaires et dans des produits pétroliers (pétroles bruts, fractions pétrolières, produits de raffinage, ...), simultanément à la quantification des hydrocarbures et du carbone. Le temps d'analyse en condition standard peut être de l'ordre de quatre-vingt dix minutes.

L'industrie pétrolière se tourne de plus en plus vers la production de pétroles bruts non conventionnels, qui sont plus riches en soufre que les pétroles conventionnels. Par ailleurs, les contraintes de teneur en soufre des produits de raffinage sont de plus en plus drastiques.

Pour ces raisons, il est crucial de savoir quantifier le soufre dans les produits pétroliers et dans les roches des systèmes géologiques pétroliers, et de le caractériser le plus finement possible.

Or, ces mesures fines sont délicates, voire impossibles dans certains cas.

Par exemple, il est impossible de définir les différentes formes moléculaires de soufre organique dans les composés organiques contenant plus de 40 atomes de carbone, qui sont pourtant les composés les plus soufrés des pétroles. D'autre part, discriminer le soufre organique du soufre minéral dans une roche nécessite, avec les moyens actuellement connus, plusieurs opérations. Une méthode et un dispositif pour la caractérisation et la quantification de soufre dans un échantillon géologique sont connus de WO2005/111603.

La présente invention permet d'apporter des informations sur les types de soufre, même dans un pétrole très lourd, et permet de discriminer le soufre organique du soufre minéral dans une roche. Elle a pour objectif principal d'être appliquée dans les domaines suivants :

### Exploration pétrolière :

- pour aider à caractériser le type de matière organique,
- pour identifier la qualité de l'huile en termes de soufre,
- pour rechercher une corrélation entre le pétrole et la roche mère,
- pour indiquer un niveau de biodégradation, phénomène qui a pour conséquence de concentrer le soufre.

### Production pétrolière :

- pour évaluer le risque de production d'H₂S selon le type de soufre en présence, dans le cadre de la récupération assistée avec des procédés thermiques.

### Raffinage:

- dans le cadre de la désulfuration des huiles.

Ainsi, la présente invention concerne une méthode pour la caractérisation et la quantification du soufre dans un échantillon de roches sédimentaires ou de produits pétroliers, dans laquelle on effectue les étapes suivantes:
- on chauffe ledit échantillon dans un four de pyrolyse en atmosphère non oxydante,
- on tranfère les résidus de pyrolyse dudit échantillon dans un four d'oxydation et on mesure en continu la quantité de SO₂ contenue dans les effluents résultants de ladite chauffe oxydante,
- on oxyde une partie des effluents de pyrolyse et on mesure en continu la quantité de SO₂ contenue dans ladite partie après oxydation.

Selon une variante de la méthode, on peut mesurer:
- les quantités de produits hydrocarbonés, de CO et de CO₂ contenues dans les effluents de pyrolyse,
- les quantités de CO et de CO₂ contenues dans les effluents résultants de ladite chauffe oxydante.

La température du four de pyrolyse peut être comprise entre 60 et 800°C.

La température du four d'oxydation peut être comprise entre 100 et 1300°C.

On peut oxyder ladite partie des effluents de pyrolyse dans un four comportant un catalyseur.

L'invention concerne également un dispositif pour la caractérisation et la quantification du soufre dans un échantillon de roches sédimentaires ou de produits pétroliers, comportant:
- un four de pyrolyse dudit échantillon en atmosphère non oxydante,
- des moyens de tranfert des résidus de pyrolyse dudit échantillon dans un four d'oxydation,
- des moyens de mesure en continu de la quantité de SO₂ contenue dans les effluents résultants de ladite chauffe oxydante,
- des moyens d'oxydation d'une partie des effluents de pyrolyse
- des moyens de mesure en continu de la quantité de SO₂ contenue dans ladite partie après oxydation.

Le dispositif peut comporter des moyens de distribution selon trois voies des effluents de pyrolyse.

Les moyens de distribution peuvent être chauffés à une température comprise entre 400 et 600°C.

Une des voies peut conduire l'effluent de pyrolyse dans lesdits moyens d'oxydation de façon à oxyder les composés soufrés en SO₂.

Les moyens d'oxydation d'une partie des effluents de pyrolyse peuvent comporter un four, un catalyseur et un moyen d'introduction d'air.

Une des voies peut conduire l'effluent de pyrolyse dans des moyens de mesure du CO et CO₂.

Une des voies peut conduire l'effluent de pyrolyse dans des moyens de mesure des composés hydrocarbonés.

La méthode selon l'invention repose donc sur la mesure des gaz soufrés émis par un échantillon qui est soumis à une pyrolyse, puis à une oxydation.
1. Phase de pyrolyse: L'échantillon est pyrolysé selon un programme de températures prédéfinies, dans un four balayé par un flux de gaz non oxydant. Une partie des effluents de pyrolyse est entraînée vers un détecteur à ionisation de flamme où les hydrocarbures sont quantifiés. Une autre partie est entraînée vers un détecteur de CO₂ et de CO. Une troisième partie est oxydée dans un four d'oxydation, en présence d'air et éventuellement d'un catalyseur. Là, les gaz soufrés sont oxydés en SO₂. Ce SO₂ est ensuite mesuré en continu, par exemple par un spectrophotomètre UV ou IR. On obtient ainsi une mesure du SO₂ en fonction de la température de pyrolyse et du temps.
2. Phase d'oxydation (oxydation à chaud): L'échantillon ayant subi l'étape de pyrolyse, est transféré depuis le four de pyrolyse vers un four d'oxydation. Le résidu y est oxydé selon un programme de températures prédéfinies, sous un flux d'air. Les effluents d'oxydation sont entraînés vers des moyens de détection de SO₂, de CO et CO₂ pour une mesure en continu de ces gaz. On obtient ainsi une mesure du SO₂ en fonction de la température d'oxydation et du temps.

Caractérisation du soufre: A l'issue de ces traitements thermiques, on dispose de deux profils de SO₂, le premier en fonction de la température de pyrolyse et le second en fonction de la température d'oxydation. Chaque profil de SO₂ comporte différents pics et est identifiable par le nombre de ces pics, leur température de sommet de pic, leur forme et leur aire. L'ensemble des deux profils constitue donc une empreinte unique caractérisant le soufre de l'échantillon. Avec cette empreinte, on peut notamment discriminer différents types de composés soufrés, comme un soufre organique "labile", un soufre organique "très labile", un soufre organique "réfractaire" et le soufre issu de la pyrite FeS₂.

Quantification de la teneur en soufre: L'aire des pics de SO₂, ramenée à celle d'un échantillon de référence dont on connaît la teneur en soufre, permet de déduire la teneur en soufre de l'échantillon analysé. On quantifie ainsi expérimentalement sa teneur en soufre, dit de pyrolyse, qui a été libéré au cours de la pyrolyse, et sa teneur en soufre dit d'oxydation, qui a été libéré au cours de l'oxydation du résidu de pyrolyse. La somme des deux teneurs est égale à la teneur en soufre total.

La présente invention procure les avantages suivants :
■ Types d'échantillons variés:
   Ce procédé permet d'étudier une vaste gamme de types d'échantillons, tels que :
      - les roches mères,
      - les kérogènes,
      - les charbons,
      - les roches réservoirs,
      - les pétroles bruts,
      - les fractions pétrolières telles que les asphaltènes,
      - les distillats pétroliers,
      - en particulier, il est bien adapté aux produits organiques lourds.
         ■ Quantité faible d'échantillon:
   Il n'est nécessaire pour l'analyse que de quelques milligrammes pour les liquides et de quelques dizaines de milligrammes pour les roches.
      ■ Durée brève d'analyse:
   Cette durée peut être de soixante à quatre-vingt dix minutes selon l'échantillon analysé.
      ■ Facilité d'utilisation du dispositif:
   Une fois l'échantillon chargé, l'automate prend en charge toutes les étapes.
      ■ Information inédite sur le type de soufre:
   En plus de quantifier la teneur en soufre total, la méthode permet de discriminer différents types de soufre, ce qui est difficile et long par d'autres techniques (Chromatographie en phase gazeuse bidimensionnelle, Spectroscopie de structure près du front d'absorption de rayons X (XANES)...), voire impossible selon le type de soufre :
      - soufre organique "très labile"
      - soufre organique "labile"
      - soufre non pyrolysable appelé soufre "réfractaire"
      - soufre pyritique
      - sulfates
   ■ Obtention d'une empreinte unique du soufre pour chaque échantillon.

La présente invention sera mieux comprise et ses avantages apparaîtront plus clairement à la lecture de la description suivante, nullement limitative, d'un mode de réalisation et d'exemples de mise en oeuvre, illustrés par les figures ci-après annexées, parmi lesquelles:
- la figure 1 illustre schématiquement le dispositif selon l'invention,
- la figure 2-a montre, dans le cas d'un pétrole brut, un exemple d'enregistrement du SO₂ libéré au cours de la pyrolyse,

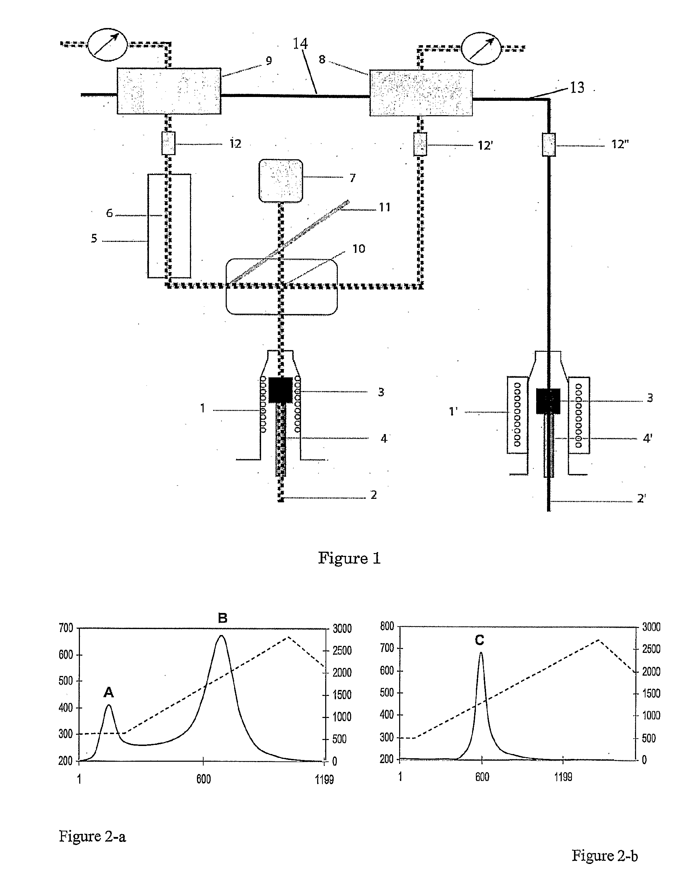

- la figure 2-b montre, dans le cas d'un pétrole brut, un exemple d'enregistrement du SO₂ libéré au cours l'oxydation,

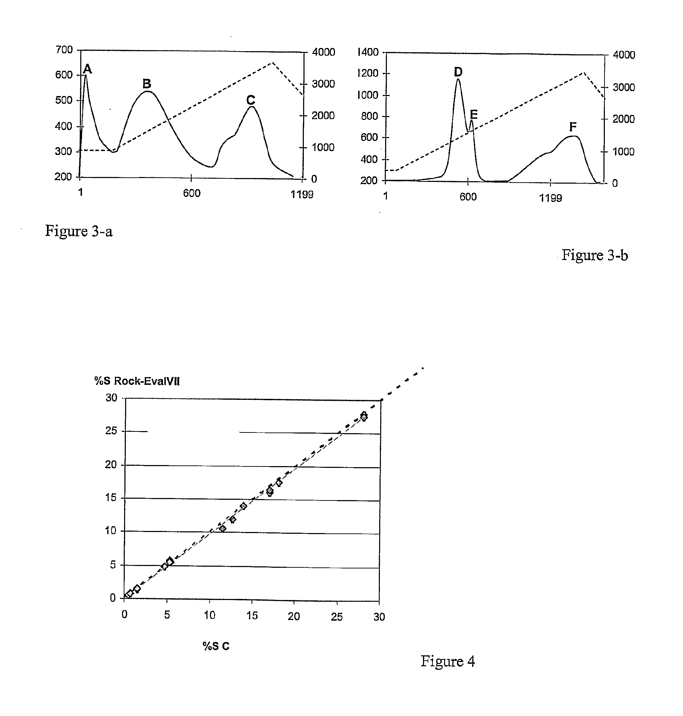

- la figure 3-a montre, dans le cas d'une roche mère, un exemple d'enregistrement du SO₂ libéré au cours de la pyrolyse,
- la figure 3-b montre, dans le cas d'une roche mère, un exemple d'enregistrement du SO₂ libéré au cours de l'oxydation
- la figure 4 montre les teneurs en soufre total de différents types d'échantillons confondus, huiles, huiles lourdes, kérogènes, composés soufrés purs, mesurée avec cette invention (en ordonnée) et par coulométrie (en abscisse).

Le dispositif mettant en oeuvre la méthode se compose principalement de trois fours et de trois détecteurs.

Deux fours sont utilisés pour le traitement thermique de l'échantillon: l'un, balayé par un flux de gaz inerte, est destiné à la pyrolyse de l'échantillon, l'autre, balayé par un flux d'air ou d'oxygène, est destiné à l'oxydation du résidu de pyrolyse.

Un troisième four est dédié à l'oxydation d'une fraction des effluents de pyrolyse.

Les trois détecteurs sont: un détecteur à ionisation de flamme (FID) pour les hydrocarbures provenant de la pyrolyse, un spectrophotomètre infrarouge (IR) pour le CO et le CO₂ provenant de la pyrolyse et de l'oxydation, et un spectrophotomètre ultraviolet (UV) ou infrarouge (IR) pour le SO₂ provenant de la pyrolyse et de l'oxydation.

La figure 1 illustre une réalisation du dispositif selon l'invention. Un four de pyrolyse 1 chauffe l'échantillon entre 60°C et 800°C, avec une programmation de température prédéfinie. La vitesse de chauffe est comprise entre 1°C/min et 50°C/min. Le four est balayé par un gaz inerte, par exemple de l'azote, avec un débit variant de 50 ml/min à 200 ml/min, qui entraîne les effluents de pyrolyse vers les analyseurs. L'azote est amené dans le four par une tubulure 2. La nacelle 3 contient l'échantillon. Le four peut être fait en inox, en alumine, porcelaine, quartz ou autre matériau adéquat. La nacelle 3 peut être en inox. Cependant, dans le cas de l'analyse du soufre et pour les oxydations à haute température, la nacelle sera de préférence en alumine ou en porcelaine, afin d'éviter la rétention de soufre sur ses parois et pour qu'elle résiste aux hautes températures. Cette nacelle est introduite dans le four de pyrolyse au moyen d'un piston 4. Celui-ci peut être fait en inox, mais sera de préférence en alumine ou en porcelaine, pour résister à l'oxydation aux fortes températures.

Un four d'oxydation 1' chauffe le résidu de pyrolyse entre 100°C et 1300°C, avec une programmation de température prédéfinie. Le four est balayé par de l'air ou de l'oxygène avec un débit constant compris entre 50 ml/min et 200 ml/min. Ce gaz vecteur est amené dans le four par la tubulure 2', et entraîne les effluents d'oxydation vers les analyseurs. La nacelle 3 contient l'échantillon après pyrolyse. Elle est introduite dans le four d'oxydation au moyen d'un piston 4'.

Un four d'oxydation des effluents de pyrolyse 5 se situe entre le four de pyrolyse et l'analyseur de SO₂. Il est destiné à transformer les effluents de pyrolyse soufrés en SO₂. L'oxydation se produit à une température constante comprise entre 500°C et 1000°C, en présence d'air ou d'oxygène. Ce four, peut fonctionner selon sa température, avec un catalyseur d'oxydation 6, par exemple du trioxyde de tungstène (WO₂), ou sans catalyseur.

Un diviseur des flux de gaz 10, chauffé entre 400°C et 600°C, est situé en sortie du four de pyrolyse 1. Il est associé à deux pompes et deux débitmètres massiques, et il permet de diviser le flux de gaz qui sort du four de pyrolyse 1 selon trois voies :
- une dirigée vers le détecteur FID 7;
- une seconde dirigée vers le spectromètre infrarouge 8;
- une troisième orientée vers le spectromètre infrarouge, ou ultraviolet 9, après passage dans le four d'oxydation 5.

Une tubulure 11, située entre le diviseur de flux 10 et le four d'oxydation des effluents de pyrolyse 5, permet d'amener de l'air ou de l'oxygène qui va servir à l'oxydation des effluents de pyrolyse.

Un système électronique de régulation des débits, associé à deux pompes, permet de contrôler les débits de gaz vers les détecteurs.

Le détecteur à ionisation de flamme 7 (FID) mesure les effluents de pyrolyse hydrocarbonés. Ceux-ci sont amenés par le courant de gaz inerte, dont le débit peut être compris entre 20 ml/min et 70 ml/min.

Le spectrophotomètre IR 8 analyse le CO, le CO₂.

Le spectrophotomètre IR ou UV 9, analyse le SO₂.

Un piège à eau 12, pouvant contenir du perchlorate de magnésium Mg(ClO₄)₂, est placé en sortie du four d'oxydation des effluents de pyrolyse 5.

Un piège à eau 12', pouvant contenir de la driérite, est situé entre le diviseur de flux 10 et le détecteur 8 de CO et CO₂.

Un piège à eau 12", pouvant contenir du perchlorate de magnésium, est situé en sortie du four d'oxydation 1'.

Les effluents du four d'oxydation 1' sont conduits vers les détecteurs 8 et 9, respectivement de CO, CO₂ et SO₂, par les canalisations référencées 13 et 14.

### Fonctionnement du dispositif

### 1. Phase de pyrolyse

On détaille ici la phase de pyrolyse, en se référant à la figure 1 qui représente schématiquement les éléments en liaison avec le four de pyrolyse 1.

On place dans la nacelle 3 un échantillon de sédiment géologique, ou d'un produit pétrolier, par exemple un pétrole brut, une fraction d'huile ou un distillat pétrolier. Selon le type d'échantillon, la masse nécessaire à l'analyse est la suivante:

| | |
|---|---|
| Roches | 20 à 100 mg |
| Kérogènes et charbons | 2 à 20 mg |
| Pétroles, huiles, distillats pétroliers | 2 à 10 mg |

La nacelle 3 est introduite dans le four de pyrolyse 1 au moyen du piston automatisé 4. Un gaz inerte (azote, hélium, ...) est admis dans le four avec un débit compris entre 50 ml/min et 200 ml/min, par l'intermédiaire de la tubulure 2. Ce gaz, appelé gaz vecteur, balaie le four et entraîne les effluents qui y sont générés pendant la pyrolyse.

Le four 1 est chauffé entre 60°C à 800°C, avec un programme d'élévation de la température prédéfini. La phase d'accroissement de la température se fait à une vitesse constante, généralement comprise entre 1°C/min et 50°C/min. Les effluents générés sont continuellement balayés par le gaz vecteur et entraînés hors du four vers le diviseur de flux de gaz 10.

Les effluents sont divisés en trois parties, dont le débit est régulé et contrôlé par des dispositifs électroniques :
∘ vers le FID 7, où les composés hydrocarbonés sont mesurés,
∘ vers le spectrophotomètre infrarouge 8, où le CO et le CO₂ sont mesurés,
∘ vers le four d'oxydation des effluents de pyrolyse 5. Avant d'atteindre ce four, le gaz est mélangé à un flux d'air ou d'oxygène amené par la tubulure 11. Ce mélange gazeux pénètre dans le four d'oxydation 5 qui est chauffé à une température constante comprise entre 500°C et 1000°C. Ce four peut, selon la température retenue, contenir un catalyseur d'oxydation, tel que le trioxyde de tungstène. Les composés soufrés contenus dans le gaz y sont transformés majoritairement en SO₂.

Les gaz passent ensuite au travers d'un piège à eau 12, composé par exemple de perchlorate de magnésium Mg(ClO₄)₂. La majeure partie de l'eau contenue dans le gaz y est retenue.

Les gaz arrivent au détecteur 9 adapté à mesurer en continu le SO₂.

### 2. Phase d'oxydation

On détaille ici la phase d'oxydation, en se référant à la figure 1.

A la fin de la phase de pyrolyse, la nacelle 3 est transférée par un automate (non représenté) depuis le four de pyrolyse 1 vers le four d'oxydation 1'.

Le four d'oxydation 1' est chauffé en suivant un programme d'élévation de la température, depuis 100°C jusqu'à une température finale qui peut atteindre 1300°C. Cette température finale est ajustée selon le type d'échantillon à étudier (huile, roche, etc..). La phase d'augmentation en température se fait à une vitesse constante, généralement comprise entre 1°C/min et 50°C/min. Pendant la chauffe, de l'air ou de l'oxygène est admis dans le four 1' par l'intermédiaire de la tubulure 2', avec un débit compris entre 50 ml/min et 200 ml/min. Ce gaz, appelé gaz vecteur, balaie le four et entraîne continuellement les effluents qui sont générés par oxydation.

Au cours de cette étape, le soufre restant après pyrolyse est oxydé en SO₂. De même, le carbone restant après pyrolyse est oxydé en CO et CO₂.

Les effluents sont entraînés hors du four et percolent au travers du piège à eau 12", où la majeure partie de l'eau contenue dans le gaz est retenue. Le SO₂, le CO et le CO₂ sont mesurés en continu en fonction du temps avec le détecteur spécifique à chaque espèce : le spectromètre 8 pour le CO et le CO₂ et le spectromètre 9 pour le SO₂.

Pour obtenir un résultat quantitatif, un calibrage du système est nécessaire.

### Exemples d'application

On présente ici deux exemples d'application, qui permettent de mieux comprendre le type d'information sur le soufre qui est fourni par l'invention. Un exemple porte sur un pétrole brut lourd et l'autre porte sur une roche mère type contenant de la pyrite et des sulfates.

La figure 2a présente le signal SO₂ enregistré au cours de la pyrolyse et la figure 2b présente le signal SO₂ enregistré au cours de l'oxydation, d'un échantillon type de pétrole brut lourd. L'axe des abscisses représente le temps en secondes. L'axe des ordonnées de gauche représente la température dans le four. L'axe des ordonnées de droite représente la quantité en milligrammes de SO₂ mesurée par seconde. Pendant la pyrolyse, le pétrole est soumis à une température de 300°C pendant 5 minutes, puis à une température croissant à la vitesse de 25°C/min jusqu'à environ 650°C (courbe pointillée). Puis, pendant l'oxydation (figure 2-b), le résidu de pyrolyse est soumis à une température constante de 300°C pendant 1 minute puis à une température croissant de 300°C à 750°C à la vitesse de 25°C/min (courbe pointillée).
Sur la figure 2-a, on distingue deux pics:
- le pic A, qui est issu de composés organiques soufrés très labiles contenus dans le pétrole brut,
- le pic B, qui est issu de composés organiques soufrés labiles contenus dans le pétrole brut.

Sur la figure 2-b, le pic C correspond au SO₂ issu de composés organiques soufrés réfractaires.
Les figures 3-a et 3-b présentent les signaux SO₂ types que l'on peut obtenir avec l'invention sur une roche mère contenant du soufre sous différentes formes: soufre organique, contenu dans le kérogène et dans l'huile, soufre de pyrite et soufre de sulfates. Pendant la pyrolyse (figure 3-a), la roche mère est soumise à une température de 300°C pendant 5 minutes, puis à une température croissant à la vitesse de 25°C/min jusqu'à environ 650°C (courbe pointillée). Pendant l'oxydation (figure 3-b), le résidu de pyrolyse est soumis à une température constante de 300°C pendant 1 minute puis à une température croissant de 300°C à 1200°C, à la vitesse de 25°C/min (courbe pointillée).
La figure 3-a montre trois pics :
- le pic A, qui est issu de composés organiques soufrés très labiles
- le pic B, qui est issu de composés organiques soufrés labiles
- le pic C, qui est issu de la pyrite.
La figure 3-b montre trois pics :
- le pic D, qui est issu de composés organiques soufrés réfractaires
- le pic E, qui est issu de la pyrite
- le pic F, qui est issu des sulfates.

### Comparaison des mesures de teneur en soufre à l'aide de la présente invention et de la mesure par coulométrie:

La teneur en soufre a été mesurée sur différents types d'échantillons, d'une part avec cette invention et d'autre part par coulométrie. La coulométrie est fréquemment utilisée pour quantifier le soufre dans les roches et dans les huiles. Elle nous fournit ici une mesure de référence.

Les résultats, présentés sur la figure 4, ont été obtenus sur des échantillons aussi variés que des huiles contenant du soufre entre 0,5% et 5% poids, des roches et des kérogènes contenant du soufre entre 10% et 20% poids, et des polymères soufrés contenant du soufre entre 15% et 30% poids. L'axe des abscisses représente la teneur massique en soufre mesurée par coulométrie. L'axe des ordonnées représente la teneur massique en soufre mesurée avec cette invention. Les résultats montrent une très bonne adéquation entre les deux techniques, sur une gamme de teneur en soufre de 0,5% à 30% poids.

## Revendications

1. Méthode pour la caractérisation et la quantification du soufre dans un échantillon de roches sédimentaires ou de produits pétroliers, dans laquelle on effectue les étapes suivantes:
- on chauffe ledit échantillon dans un four de pyrolyse (1) en atmosphère non oxydante,
- on tranfère les résidus de pyrolyse dudit échantillon dans un four d'oxydation (1') et on mesure en continu la quantité de SO₂ contenue dans les effluents résultants d'une chauffe oxydante dans ledit four d'oxydation,
- on oxyde une partie des effluents résultant de la pyrolyse en atmosphère non-oxydante et on mesure en continu la quantité de SO₂ contenue dans ladite partie après oxydation.

2. Méthode selon la revendication 1, dans laquelle on mesure:
- les quantités de produits hydrocarbonés, de CO et de CO₂ contenues dans les effluents de pyrolyse,
- les quantités de CO et de CO₂ contenues dans les effluents résultants de ladite chauffe oxydante.

3. Méthode selon l'une des revendications 1 ou 2, dans laquelle la température du four de pyrolyse est comprise entre 60 et 800°C.

4. Méthode selon l'une des revendications 1 à 3, dans laquelle la température du four d'oxydation est comprise entre 100 et 1300°C.

5. Méthode selon l'une des revendications précédentes, dans laquelle on oxyde ladite partie des effluents de pyrolyse dans un four comportant un catalyseur.

6. Dispositif pour la caractérisation et la quantification du soufre dans un échantillon de roches sédimentaires ou de produits pétroliers, comportant:
- un four de pyrolyse (1) dudit échantillon en atmosphère non oxydante,
- des moyens de tranfert des résidus de pyrolyse dudit échantillon dans un four d'oxydation (1'),
- des moyens d'oxydation (5) d'une partie des effluents résultant de la pyrolyse en atmosphère non-oxydante,
- des moyens de mesure en continu (9) de la quantité de SO₂ contenue dans ladite partie après oxidation, et de la quantité de SO2 contenue dans les effluents résultants d'une chauffe oxydante dans ledit four d'oxydation.

7. Dispositif selon la revendication 6, comportant des moyens de distribution (10) selon trois voies des effluents de pyrolyse.

8. Dispositif selon la revendication 7, dans lequel lesdits moyens de distribution sont chauffés à une température comprise entre 400 et 600°C.

9. Dispositif selon l'une des revendications 7 à 8, dans lequel une des voies conduit l'effluent de pyrolyse dans lesdits moyens d'oxydation (5) de façon à oxyder les composés soufrés en SO₂.

10. Dispositif selon l'une des revendications 6 à 9, dans lequel les moyens d'oxydation d'une partie des effluents de pyrolyse comportent un four, un catalyseur et un moyen d'introduction d'air.

11. Dispositif selon l'une des revendications 7 à 10, dans lequel une des voies conduit l'effluent de pyrolyse dans des moyens de mesure (8) du CO et CO₂.

12. Dispositif selon l'une des revendications 7 à 11, dans lequel une des voies conduit l'effluent de pyrolyse dans des moyens de mesure des composés hydrocarbonés.

## Patentansprüche

1. Verfahren zur Charakterisierung und Quantifizierung von Schwefel in einer Probe aus Sedimentgestein oder Erdölerzeugnissen, wobei die folgenden Schritte durchgeführt werden:
- Erhitzen der Probe in einem Pyrolyseofen (1) in nicht oxidierender Atmosphäre,
- Übertragen der Pyrolyserückstände der Probe in einen Oxidationsofen (1') und kontinuierliches Messen der Menge an SO₂, die in den Abflüssen enthalten ist, die aus dem oxidierenden Erhitzen resultieren,
- Oxidieren eines Teils der Abflüsse, die aus der Pyrolyse in nicht oxidierender Atmosphäre resultieren, und kontinuierliches Messen der enge an SO₂, die in dem Teil nach der Oxidation enthalten ist.

2. Verfahren nach Anspruch 1, wobei gemessen werden:
- die Mengen an Kohlenwasserstofferzeugnissen, an CO und an CO₂, die in den Pyrolyseabflüssen enthalten sind,
- die Mengen an CO und CO₂, die in den Abflüssen enthalten sind, die aus dem oxidierenden Erhitzen resultieren.

3. Verfahren nach einem der Ansprüche 1 oder 2, wobei die Temperatur des Pyrolyseofens im Bereich zwischen 60 und 80 °C liegt.

4. Verfahren nach einem der Ansprüche 1 bis 3, wobei die Temperatur des Oxidationsofens im Bereich zwischen 100 und 1.300 °C liegt.

5. Verfahren nach einem der vorhergehenden Ansprüche, wobei der Teil der Pyrolyseabflüsse in einem Ofen, der einen Katalysator umfasst, oxidiert wird.

6. Vorrichtung zur Charakterisierung und Quantifizierung von Schwefel in einer Probe aus Sedimentgestein oder Erdölerzeugnissen, umfassend:
- einen Ofen zum Pyrolysieren (1) der Probe in nicht oxidierender Atmosphäre,
- Mittel zum Übertragen der Pyrolyserückstände der Probe in einen Oxidationsofen (1'),
- Mittel zum Oxidieren (5) in nicht oxidierender Atmosphäre eines Teils der Abflüsse, die aus der Pyrolyse resultieren,
- Mittel zum kontinuierlichen Messen (9) der Menge an SO₂, die nach dem Oxidieren in dem Teil enthalten ist, und der Menge an SO₂, die in den Abflüssen enthalten ist, die aus einem oxidierenden Erhitzen in dem Oxidationsofen resultieren.

7. Vorrichtung nach Anspruch 6, umfassend Mittel zum Verteilen (10) der Pyrolyseabflüsse auf drei Kanäle.

8. Vorrichtung nach Anspruch 7, wobei die Mittel zum Verteilen auf eine Temperatur im Bereich zwischen 400 und 600 °C erhitzt werden.

9. Vorrichtung nach einem der Ansprüche 7 bis 8, wobei einer der Kanäle den Pyrolyseabfluss in die Oxidationsmittel (5) leitet, um die Schwefelverbindungen zu SO₂ zu oxidieren.

10. Vorrichtung nach einem der Ansprüche 6 bis 9, wobei die Mittel zur Oxidation eines Teils der Pyrolyseabflüsse einen Ofen, einen Katalysator und ein Mittel zum Einführen von Luft umfassen.

11. Vorrichtung nach einem der Ansprüche 7 bis 10, wobei einer der Kanäle den Pyrolyseabfluss in Mittel zum Messen (8) von CO und CO₂ leitet.

12. Vorrichtung nach einem der Ansprüche 7 bis 11, wobei einer der Kanäle den Pyrolyseabfluss in Mittel zum Messen der Kohlenwasserstoffverbindungen leitet.

## Claims

1. A method for sulfur characterization and quantification in a sample of sedimentary rocks or of petroleum products, wherein the following stages are carried out:
- heating said sample in a pyrolysis oven (1) in a non-oxidizing atmosphere,
- transferring the pyrolysis residues of said sample into an oxidation oven (1') and continuously measuring the amount of SO₂ contained in the effluents resulting from oxidizing heating in said oxidation oven,
- oxidizing part of the effluents resulting from the pyrolysis in a non-oxidizing atmosphere and continuously measuring the amount of SO₂ contained in said part after oxidation.

2. A method as claimed in claim 1, wherein the following amounts are measured:
- amounts of hydrocarbon-containing products, of CO and of CO₂ contained in the pyrolysis effluents,
- amounts of CO and of CO₂ contained in the effluents resulting from said oxidizing heating.

3. A method as claimed in any one of claims 1 or 2, wherein the temperature of the pyrolysis oven ranges between 60°C and 800°C.

4. A method as claimed in any one of claims 1 to 3, wherein the temperature of the oxidation oven ranges between 100°C and 1300°C.

5. A method as claimed in any one of the previous claims, wherein said part of the pyrolysis effluents is oxidized in an oven comprising a catalyst.

6. A device for sulfur characterization and quantification in a sample of sedimentary rocks or of petroleum products, comprising:
- a pyrolysis oven (1) for heating said sample in a non-oxidizing atmosphere,
- means for transferring the pyrolysis residues of said sample into an oxidation oven (1'),
- means (5) for oxidizing part of the effluents resulting from the pyrolysis in a non-oxidizing atmosphere,
- means (9) for continuous measurement of the amount of SO₂ contained in said part after oxidation and of the amount of SO₂ contained in the effluents resulting from oxidizing heating in said oxidation oven.

7. A device as claimed in claim 6, comprising three-port distribution means (10) for the pyrolysis effluents.

8. A device as claimed in claim 7, wherein said distribution means are heated to a temperature ranging between 400°C and 600°C.

9. A device as claimed in any one of claims 7 to 8, wherein one of the ports sends the pyrolysis effluent to said oxidation means (5) so as to oxidize the sulfur compounds to SO₂.

10. A device as claimed in any one of claims 6 to 9, wherein the means for oxidizing part of the pyrolysis effluents comprise an oven, a catalyst and an air supply means.

11. A device as claimed in any one of claims 7 to 10, wherein one of the ports sends the pyrolysis effluent to CO and CO₂ measuring means (8).

12. A device as claimed in any one of claims 7 to 11, wherein one of the ports sends the pyrolysis effluent to hydrocarbon compound measuring means.
